# EUROPEAN PATENT SPECIFICATION

(11) **EP 2 191 794 B1**
(45) Date of publication and mention of the grant of the patent: **22.08.2012**
(21) Application number: 10155200.8
(22) Date of filing: 18.05.2006
(51) Int. Cl.: A61F 2/90

(54) **Medical devices**
Medizinische Vorrichtungen
Dispositifs médicaux

(30) Priority: 27.05.2005 US 140401
(43) Date of publication of application: 02.06.2010
(62) Divisional of application: 06760118.7
(73) Proprietor: Boston Scientific Limited, Christ Church (BB)
(72) Inventor: Stinson, Jonathan S., Plymouth, MN 55447 (US); Cambronne, Matthew, Mounds View, MN 55112 (US)
(74) Representative: Peterreins, Frank

(56) References cited:
- US-A1- 2002 197 178
- US-A1- 2003 060 873
- US-A1- 2004 237 282
- US-A1- 2005 075 408

## Description

### TECHNICAL FIELD

The invention relates to medical devices, such as endoprostheses (e.g., stents).

### BACKGROUND

The body includes various passageways such as arteries, other blood vessels, and other body lumens. These passageways sometimes become occluded or weakened. For example, the passageways can be occluded by a tumor, restricted by plaque, or weakened by an aneurysm. When this occurs, the passageway can be reopened or reinforced, or even replaced, with a medical endoprosthesis. An endoprosthesis is typically a tubular member that is placed in a lumen in the body. Examples of endoprostheses include stents, covered stents, and stent-grafts.

Endoprostheses can be delivered inside the body by a catheter that supports the endoprosthesis in a compacted or reduced-size form as the endoprosthesis is transported to a desired site. Upon reaching the site, the endoprosthesis is expanded, for example, so that it can contact the walls of the lumen.

The expansion mechanism may include forcing the endoprosthesis to expand radially. For example, the expansion mechanism can include the catheter carrying a balloon, which carries a balloon-expandable endoprosthesis. The balloon can be inflated to deform and to fix the expanded endoprosthesis at a predetermined position in contact with the lumen wall. The balloon can then be deflated, and the catheter withdrawn.

In another delivery technique, the endoprosthesis is formed of an elastic material that can be reversibly compacted and expanded, e.g., elastically or through a material phase transition. During introduction into the body, the endoprosthesis is restrained in a compacted condition. Upon reaching the desired implantation site, the restraint is removed, for example, by retracting a restraining device such as an outer sheath, enabling the endoprosthesis to self- expand by its own internal elastic restoring force.

When the endoprosthesis is advanced through the body, its progress can be monitored, e.g., tracked, so that the endoprosthesis can be delivered properly to a target site. After the endoprosthesis is delivered to the target site, the endoprosthesis can be monitored to determine whether it has been placed properly and/or is functioning properly. Methods of monitoring a medical device include X-ray fluoroscopy, computed tomography (CT), and magnetic resonance imaging (MRI).

Document US 2003/0060873 discloses a composite material for endoprostheses consisting of a metal matrix in which metal particles are incorporated.

### SUMMARY

Aspects of the invention feature medical devices including endoprostheses (e.g., stents, grafts, stent grafts and the like), and methods of making the medical devices. In one aspect, endoprostheses are provided that are made up, at least in part, of a composite. The composite includes a first structure having a first material and having interconnected portions that define interconnected channels. A second material is in at least some of the interconnected channels as described in claim 1. The first material and second material have different chemical compositions.

In another aspect, endoprostheses are featured having a first portion and a second portion as described in claim 1. The endoprostheses include a composite having a first structure that includes a first material and that has interconnected portions that define interconnected channels. The first structure has portions that extend into both the first portion of the endoprosthesis and the second portion of the endoprosthesis. A second material having a different chemical composition than the first material is in at least some of the interconnected channels of the portion of the first structure that extends into the first portion of the endoprosthesis as described in claim 1.

Embodiments may include one or more of the following features.

The first material and the second material may differ from each other in at least one material characteristic (e.g., density, mass absorption coefficient, yield strength, % elongation, modulus) which may affect at least one endoprosthesis parameter (e.g., radiopacity, magnetic susceptibility, expansion pressure, compression strength, axial flexibility, radial stiffness). The first and second materials each may impart different properties to the endoprosthesis. The first material and/or the second material may include a metal or an alloy. One or both of the first material and second material may include a biodegradable or biostable polymer. One or both of the first material and second material may include a high strength material, e.g., iron and alloys thereof (for example, stainless steel), cobalt and alloys thereof, titanium and alloys thereof, and nickel and alloys thereof. One or both of the first material and second material may include a radiopaque material, e.g., tantalum, rhenium, indium, platinum, gold, silver, indium, niobium, molybdenum, and alloys of any of these. One or both of the first material and second material may include molybdenum, tungsten, chromium, tantalum over carbon Trabecular Metal™ foam, and niobium over carbon Trabecular Metal™ foam. One or both of the first material and second material may include 316L stainless steel, L605, MP35N® alloy, Elgiloy®, platinum enhanced radiopaque stainless steel (PERSS), Nb-1Zr, Nioballoy, NiTi, Biodur® 108 stainless steel, zirconium, an alloy of zirconium, titanium, an alloy of titanium, biostable polymer and bioabsorbable polymer.

The channels may have the form of pores or hollow passageways, and may have a regular or an irregular cross-section. The channels may be between about 10 microns and about 10 mm in diameter at their narrowest part. At least about 50% (e.g., at least about 75%) of the channels defined by the interconnected portions of the first structure may be interconnected. Substantially all of the channels defined by the interconnected portions of the first structure may be connected to at least one channel that is open to the exterior of the first structure. Between about 10% and about 90% (e.g., between about 50% and about 90%) of the total volume of the first structure may comprise channels.

The first structure may have solid and hollow portions where the solid portions may be a continuous or discontinuous framework formed of metal and/or polymer and the hollow portions may include pores, channels, gaps, voids, and/or open areas. The first structure may be in the form of, e.g., a foam, a wire weave mesh, a plasma-sprayed deposit, a braided wire, a tangled wire, a honeycomb or any combinations thereof. The first structure may have a width, e.g., a maximum width, that is less than, e.g., between 5% and 90% of, the width or thickness of a wall of the endoprosthesis (e.g., of the width or thickness of a strut of the endoprosthesis).

The first structure is substantially encapsulated by the second material. The second material may be bonded to the first structure, e.g., mechanically bonded, adhesively bonded, and/or metallurgically bonded. The endoprosthesis may be a stent or a stent graft.

The first portion of the endoprosthesis may be disposed inwardly relative to the second portion of the endoprosthesis (e.g., the first portion may face the interior of the endoprosthesis and the second portion may face the exterior of the endoprosthesis). The composite may further include a third material in at least some of the interconnected channels of the portion of the first structure that extends into the second portion of the endoprosthesis. The first material, second material and third material may have chemical compositions different from each other. The second material may have poor solid solubility with the third material, that is to say, the second and third materials may be incapable of or poorly capable of metallurgically bonding to one another, such that the endoprosthesis may risk delamination absent the first structure.

In another aspect, methods of making endoprosthesis in accordance with any of those described above are provided as claimed in claim 12. The methods include forming a composite as claimed in claim 12 by introducing a second material into at least some interconnected channels defined by interconnected portions of a first structure, where the first structure includes a first material having a different chemical composition than the second material. The composite is used to form at least a portion of the endoprosthesis.

Embodiments may include one or more of the following features.

Second material in powdered form may be introduced into at least some of the interconnected channels in the first structure to form a dry composite, which maybe consolidated in to a composite under pressure, e.g., cold pressing, sintering or hot isostatic pressure. The first structure may be agitated, e.g., by shaking, vibrating or centrifugally rotating the first structure, which may assist in distribution of the powered second material.

Second material in molten form may be introduced into at least some of the channels in the first structure. The second material may have a lower melting temperature than the first structure, e.g., the second material may be titanium and the first structure may be made of talantum. The second material may be melted by vacuum induction skull method. The first structure may be placed in a mold and molten second material may be introduced into the mold. The first structure may be agitated, e.g., by shaking, vibrating or centrifugally rotating the first structure, which may assist in distribution of the molten second material.

A mold having hollow passages in the shape of the first structure may be formed out of second material, and first material, e.g., molten or powdered first material, may be introduced into the hollow passages. The mold may be agitated, e.g., by shaking, vibrating or centrifugally rotating the mold, which may assist in distribution of the first material. The mold may be formed by introducing molten second material into a mold having a removable core, e.g., a ceramic core, in the form of the first structure and extending to at least one edge of the mold; allowing the second material to harden removing the removable core to leave hollow passages in the hardened second material; and introducing molten first material into the hollow passages in the hardened second material. The removable core may be removed by leaching, ashing, or other suitable means.

The first structure may be made in the form of a mat having interconnected channels that is then overlayed with and bonded to a layer of second material, e.g. by annealing or diffusion bonding. The mat may be overlayed with and bonded to a layer of third material, for example, on an opposite face of the mat from the layer of second material or at a region of the mat not overlayed with second material. The third material may have a different chemical composition than the second material.

The composite may be densified into a billet, e.g., through the application of heat and isostatic pressure. The billet may be further processed to form the endoprosthesis. For example, the billet can be hot pressed, hot extruded or forged (e.g., gyratory forging machine (GFM) forged, press forged, closed-die forged) to form a rod, which may be hollowed, e.g., gun drilled, and drawn into stent tubing. The stent tubing may be laser cut to form a stent. The cut affected areas may be removed, and the stent may be finished, e.g., finely polished. A biocompatible coating may be applied to the endoprosthesis, e.g., coated on the interior surface of the endoprosthesis, exterior surface of the endoprosthesis, or over the entirety of the endoprosthesis.

Embodiments may include one or more of the following advantages.

The endoprosthesis components may be selected to provide the endoprosthesis with desirable characteristics, e.g., mechanical or physical characteristics, that may not be available from an endoprosthesis made up of a single material rather than a composite. For example, while it is often desirable for an endoprosthesis to have both high strength and sufficient radiopacity to be readily observable by a physician, e.g., during implantation, many radiopaque agents have limited solid solubility in high strength alloys. Use of composites disclosed herein may allow for combinations of high strength alloys and radiopaque agents sufficient for this purpose. Components may be selected such that other endoprosthesis parameters can be optimized, for example, the strength, stiffness, radiopacity, yield strength, ductility, magnetic susceptibility, biocompatibility, and the like. Tissue ingrowth into the endoprosthesis structure can be encouraged. Tissue ingrowth into a lumen of the endoprosthesis, e.g., a stent lumen, can be prohibited.

Materials having poor solid solubility may be layered to form an endoprosthesis having a lower risk of delamination than maybe the case absent the first structure.

Other aspects, features, and advantages will be apparent from the description of the preferred embodiments thereof and from the claims.

### DESCRIPTION OF DRAWINGS

Fig. 1 is a perspective view of an embodiment of an expanded stent.
Fig. 2 is a cross sectional view of a strut of the stent of Fig. 1, taken along the line 1-1.
Fig. 3 is a view of an embodiment of a first structure.
Fig. 4A is a view of an embodiment of a first structure.
Fig. 4B is a view of an embodiment of a first structure.
Fig. 4C is a view of an embodiment of a first structure.
Fig. 4D is a view of an embodiment of a first structure.
Fig. 4E is a view of an embodiment of a first structure.
Fig. 5 is a cross-sectional view of an embodiment of an endoprosthesis.
Fig. 6 is a cross-sectional view of an embodiment of an endoprosthesis.
Fig. 7 is a flow chart of an embodiment of a method of making a stent.
Fig. 8 is an embodiment of a mat overlayed by a layer of second material.
Fig. 9 is an embodiment of a mat overlayed by a layer of second material and a layer of third material.
Fig. 10 is a schematic for an embodiment of manufacturing of a composite.
Fig. 11 is a schematic for an embodiment of manufacturing of a composite.
Fig. 12 is a schematic for an embodiment of manufacturing of a composite.

### DETAILED DESCRIPTION

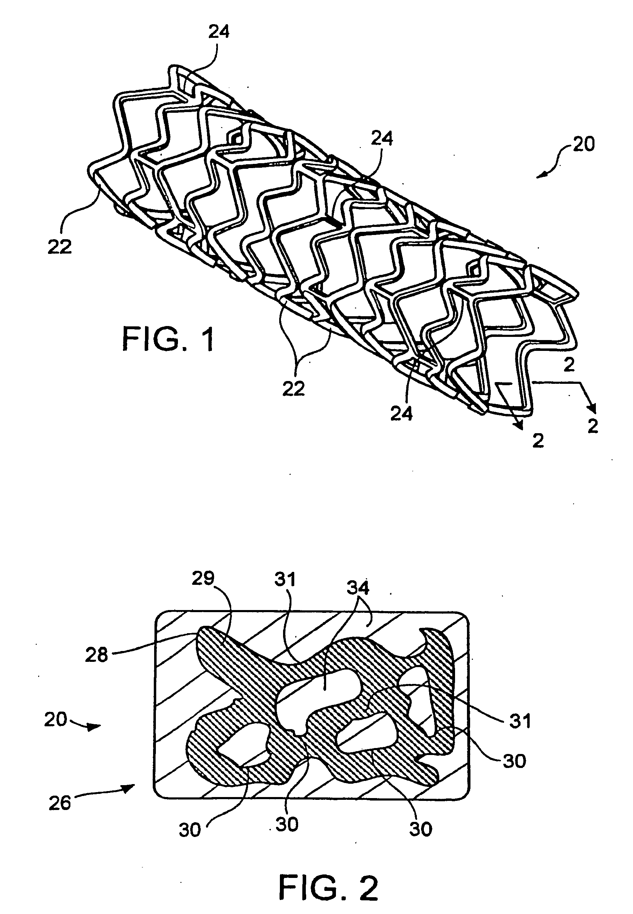

Referring to Fig. 1 , a stent 20 has the form of a tubular member defined by a plurality of bands 22 and a plurality of connectors 24 that extend between and connect adjacent bands.

During use, bands 22 may be expanded from an initial, small diameter to a larger diameter to contact stent 20 against a wall of a vessel, thereby maintaining the patency of the vessel. Connectors 24 may provide stent 20 with flexibility and conformability that allow the stent to adapt to the contours of the vessel.

Referring to Fig. 2, stent 20 includes a composite 26 that includes a first material 29 and a second material 34 having a different chemical composition than that of the first material 29. Composite 26 is made by providing a first structure 28 including first material 29 and having interconnected portions 31 defining channels 30, and introducing second material 34 into the channels 30. Second material 34 can wholly or partially fill all or some of the channels. For example, as illustrated in Fig. 2, second material 34 fills channels 30 of first structure 28, and extends around first structure 28 to encapsulate or fully cover the first structure. Second material 34 may be in at least 50% of the interconnected channels defined by the first structure (e.g., at least 60% of the interconnected channels defined by the first structure, at least 70% of the interconnected channels defined by the first structure, at least 80% of the interconnected channels defined by the first structure). Second material 34 is thus in direct contact with first structure 28 so as to form a bond with first structure 28. The bond between the first material and the second material can be a metallurgical bond having a diffusion layer between the materials, or the bond can be a mechanical bond without a diffusion layer.

In some embodiments, at least some of the channels are interconnected (e.g., at least 25% of the channels are interconnected, at least 30% of the channels are interconnected, at least 40% of the channels are interconnected, at least 50% of the channels are interconnected, at least 60% of the channels are interconnected, at least 70% of the channels are interconnected, at least 75% of the channels are interconnected, at least 80% of the channels are interconnected). The degree of interconnectivity of the channels can be measured by casting the first structure in epoxy, grinding the casting down to a planar cross-section, counting the number of channel intersections that are connected and not connected, e.g., using microscopy, and converting these values to a percentage.

In some embodiments, the interconnecting of channels is such that, when the channels are filled with second material, the second material forms a second structure that is interwoven or interlaced with the first structure. Such interweaving may result in an enhanced mechanical bond between the first structure and the second material.

The composite material is capable of providing endoprostheses with tailored physical properties (such as density, stiffness and radiopacity) and/or mechanical properties (such as yield strength and ductility). For example, a stent made of pure tantalum can have good biocompatibility and a low magnetic susceptibility that provides good visualization during magnetic resonance imaging (MRI). But in some embodiments, a pure tantalum stent may be too highly radiopaque, and as a result, visualization of the volume in the stent and of the tissue surrounding the stent during X-ray fluoroscopy or computed tomography (CT) may be obscured. To reduce the radiopacity, the stent can be made of a composite material including a component (e.g., the first structure, the second material, a third material) including tantalum and a component including a less dense material, such as titanium. Since the titanium is less dense than tantalum, the radiopacity of a sample of the composite is reduced relative to an otherwise identical sample of tantalum. At the same time, since titanium also has good biocompatibility and a low magnetic susceptibility, the composite also has good biocompatibility and MRI compatibility. Further, since titanium has high yield strength, the composite has increased strength relative to tantalum alone. Other high density materials, such as molybdenum, niobium, platinum, and their alloys, can be similarly modified as described above for tantalum. Reducing the radiopacity may be particularly beneficial for thick walled stents (such as peripheral vascular stents). As another example, the composite can also be used to increase the radiopacity of an endoprosthesis, such as by combining a component including tantalum particles with a Nitinol component. Increasing the radiopacity may be particularly beneficial for thin walled stents and may obviate the need for radiopaque markers on stents. When combining such components, either the first or the second material may be utilized to enhance any desired property. For example, either the first material or the second material can be selected to enhance the strength of the device or to enhance the radiopacity of the device.

The materials can also be selected so as to enhance the mechanical properties of the device. Without wishing to be bound by theory, it is believed that one component can be selected to increase the stiffness (tensile modulus) and/or strength (yield strength) of the composite (as compared against the strength of a similar device made only of the other material). For example, tantalum, tungsten, and/or rhenium can increase the stiffness of component including titanium or niobium, as compared against pure titanium or niobium. The enhanced mechanical properties may allow the stent to be formed with reduced wall thickness without compromising the performance of the stent. A thinner walled stent may be more easily delivered through a tortuous path, may be implanted in a smaller bodily vessel, and may allow more fluid flow through the stent.

The first structure in some embodiments includes (e.g., is manufactured from) a first material that itself can include one or more biocompatible materials with mechanical properties that allow an endoprosthesis including composite material to be compacted, and subsequently expanded to support a vessel. In some embodiments, the endoprosthesis material can have an ultimate tensile strength (UTS) of about 20-150 ksi, greater than about 10% elongation to failure, and a modulus of elasticity of about 10-60 msi. When the endoprosthesis is expanded, the material can be stretched to strains on the order of about 0.6. Examples of first materials for the first structure include stainless steel (e.g., 316L and 304L stainless steel, and an alloy including stainless steel and 5-60% by weight of one or more radiopaque elements (e.g., Pt, Ir, Au, W) (PERSS®) as described in US-2003-0018380-A1, US-2002-0144757-A1, and US-2003-0077200-A1), Nitinol (a nickel-titanium alloy), Elgiloy, L605 alloys, MP35N, titanium, titanium alloys (e.g., Ti-6A1-4V, Ti-50Ta, Ti-I0Ir), platinum, platinum alloys, chromium, chromium alloys, molybdenum, molybdenum alloys, niobium, niobium alloys (e.g., Nb-1Zr), Co-28Cr-6Mo, tantalum, and tantalum alloys. Other examples of materials are described in commonly assigned U.S.S.N. 10/672,891, filed September 26, 2993, and entitled "Medical Devices and Methods of Making Same"; and U.S.S.N. 11/035,316, filed January 3, 2005, and entitled "Medical Devices and Methods of Making Same". Other materials include elastic biocompatible metal such as a superelastic or pseudo-elastic metal alloy, as described, for example, in Schetsky, L. McDonald, "Shape Memory Alloys", Encyclopedia of Chemical Technology (3rd ed.), John Wiley & Sons, 1982, vol. 20. pp. 726-736; and commonly assigned U.S.S.N. 10/346,487, filed January 17, 2003.

In some embodiments, the first structure includes one or more materials that enhance visibility by MRI. Examples of MRI materials include non-ferrous metals (e.g., copper, silver, platinum, or gold) and non-ferrous metal-alloys containing paramagnetic elements (e.g., dysprosium or gadolinium) such as terbium-dysprosium, dysprosium, and gadolinium. Alternatively or additionally, the first structure can include one or more materials having low magnetic susceptibility to reduce magnetic susceptibility artifacts, which during imaging can interfere with imaging of tissue, e.g., adjacent to and/or surrounding the stent. Low magnetic susceptibility materials include those described above, such as tantalum, platinum, titanium, niobium, copper, chromium, molybdenum, and alloys containing one or more of these elements.

The second material in some embodiments can include one or more biocompatible materials, for example, those described above with regard to the first structure, provided the chemical composition(s) of the first material is different from the chemical composition(s) of the second material. In embodiments in which the second material includes one or more radiopaque materials to enhance radiopacity, the particles may include metallic elements having atomic numbers greater than 26 (e.g., greater than 43), and/or a density greater than about 9.9 g/cc. hi certain embodiments, the radiopaque material is relatively absorptive of X-rays, e.g., having a linear attenuation coefficient of at least 25 cm'1, e.g., at least 50 cm"1, at 100 keV. Some radiopaque materials include tantalum, platinum, iridium, palladium, hafnium, tungsten, gold, ruthenium, and rhenium. The radiopaque material can include an alloy, such as a binary, a ternary or more complex alloy, containing one or more elements listed above with one or more other elements such as iron, nickel, cobalt, or titanium. Examples of alloys including one or more radiopaque materials are described in U.S. Application Publication US-2003-0018380-A1; US-2002-0144757-A1; and US-2003- 0077200-A1. Alternatively or additionally, in embodiments in which the second material includes one or more components or materials to enhance MRI visibility, the second material may include an oxide or a carbide layer of dysprosium or gadolinium (e.g., Dy203 or Gd203); a superparamagnetic material, such as Fe304, CoFe204, MnFe204, or MgFe204; and/or transition metal oxides (e.g., oxides of Fe, Co, Ni). Thus, the chemical composition of the second material need not be limited to metallic materials (e.g., metal and alloys), but the particles may include non-metallic compounds, such as ceramics, for example, oxides (e.g., aluminum oxide), carbides (e.g., silicon carbide), and nitrides (e.g., titanium nitrides), as further illustrated by the exemplary materials listed above.

The relative quantities of first material and second material in the composite can vary depending on the particular materials utilized and the endoprosthesis parameter or parameters sought. For example, a composite may incorporate between about 1 and about 50 volume percent of a radiopaque material where radiopacity is sought. In certain embodiments, the radiopaque material is a high density material, e.g., platinum, that typically need be present in lower amounts, e.., between about 1 and about 30 volume percent. In certain embodiments, the radiopaque material is a lower density material, for example, niobium, and is present in somewhat higher amounts, e.g., between about 20 and about 50 volume percent.

As another example, a composite may include a material designed to increase the stiffness of the endoprosthesis at between about 20 and about 80 volume percent. For example, stainless steel, which has stiffness similar to current stents, can be incorporated at between, e.g., about 60 and about 80 volume percent, whereas a material having a high modulus, e.g., iridium, can be included in amounts ranging, e.g., from about 20 to about 40 volume percent.

As another example, a composite may include a material designed to increase the yield strength of the composite at between about 20 and about 80 volume percent. For example, stainless steel, which has a yield strength similar to current stents, can be incorporated at between, e.g., about 60 and about 80 volume percent, whereas a material having a high yield strength, e.g., Ti-6A1-4V, can be included in amounts ranging, e.g., from about 20 to about 40 volume percent.

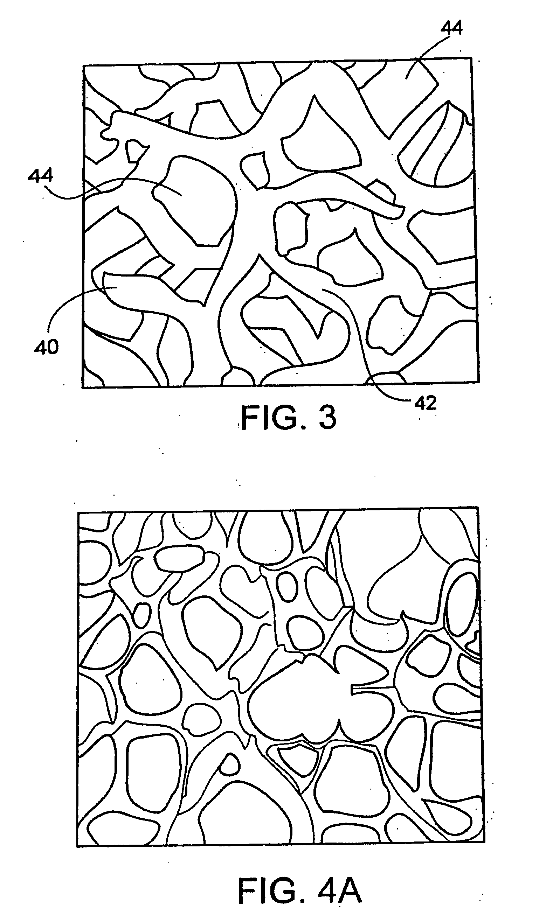

Fig. 3 shows a three-dimensional view of an embodiment of a first structure 40 absent any second material. In this embodiment, the first structure 40 is made up of a series of interconnected portions 42 that define interconnected channels 44. For example, a first channel and a second channel may be connected together to define a plane, and at least one other channel (at least two channels, at least three channels, at least four channels, at least five channels) may be directly connected to the first channel or the second channel and extend transversely to the plane. At least some of the channels may be interconnected to more than one channel (e.g., maybe interconnected to at least two channels, at least three channels, at least four channels, at least five channels), and may be connected to multiple channels extending in at least two different directions (e.g., at least three different directions, at least four different directions, at least five different directions).

Substantially all (for example, 80%, 90%, 95%) of the channels 44 are interconnected to each other. Further, substantially all of the channels defined by the interconnected portions of the first structure may be connected to at least one channel that is open to the exterior of the first structure, not illustrated, to permit or facilitate the filling of the open volumes with second (or third, fourth, etc.) material as discussed further below. In some embodiments, the majority of the channels are themselves open to the exterior of the first structure, for example, 50% of the channels are open to the exterior of the first structure, 75% of the channels are open to the exterior of the first structure, 80% of the channels are open to the exterior of the first structure, 90% of the channels are open to the exterior of the first structure, 95% of the channels are open to the exterior of the first structure.

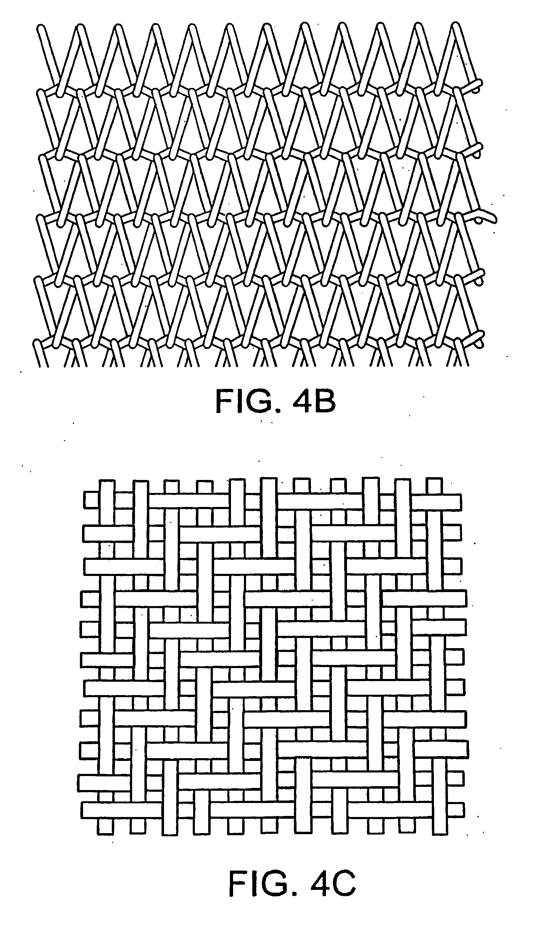

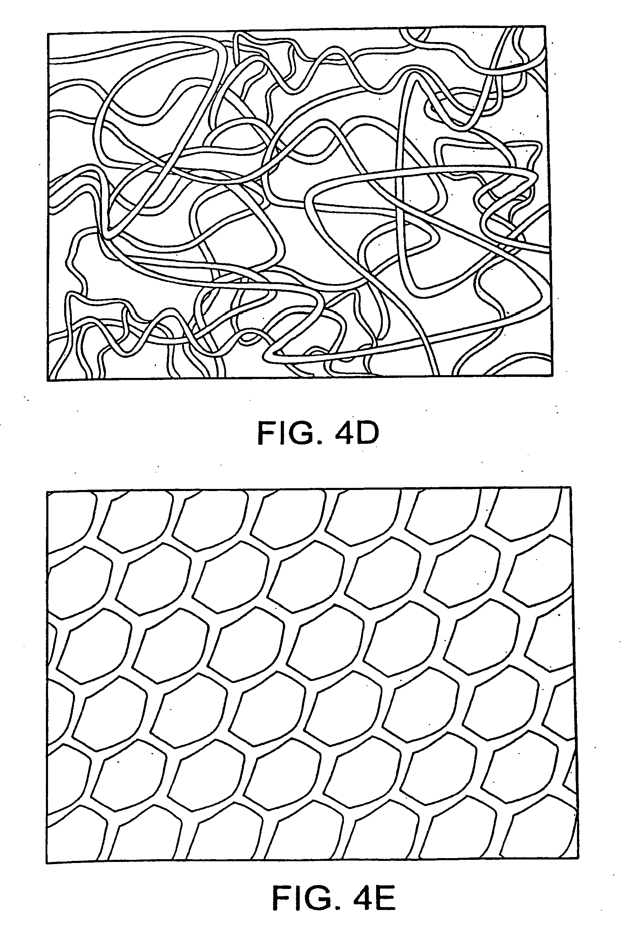

Figs. 4A-4E show certain embodiments of the first structure. Fig 4A shows a foam, which can be, for example, a Trabecular Metal™ foam (a material composed of tantalum on a graphite scaffold) in which channels interconnect with each other in a three-dimensional fashion. Fig. 4B shows a single layer of a wire weave mesh; typically, at least two such layers are be overlaid to form a three-dimensional network of interconnected channels. A single layer of a braided wire is illustrated in Fig. 4C; again, braided wires are typically arranged to provide a three-dimensional network of interconnected channels by, or example, overlaying at least two such layers. Fig. 4D shows a tangled wire having a three-dimensional network of interconnected channels.
Fig. 4E shows a single layer of a honeycomb structure, which may also typically be arranged to provide a three-dimensional network of interconnected channels. Forms employing combinations of these are also possible. The first structure may also utilize any other form having a substantially continuous structure defining interconnected channels, or any combination of such forms.

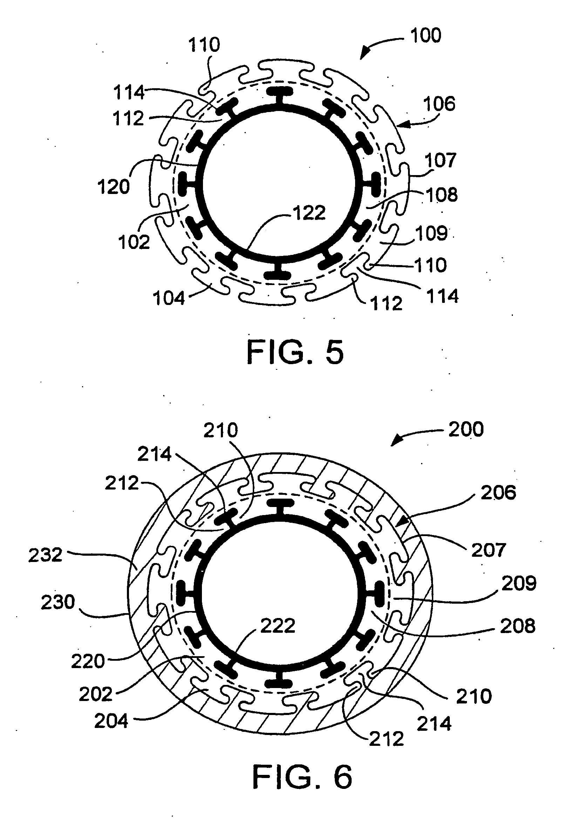

An embodiment of an endoprosthesis is illustrated in Fig. 5. Generally cylindrical endoprosthesis 100 includes a first portion 102 which makes up the interior portion of endoprosthesis 100 and a second portion 104 which makes up the outer portion of endoprosthesis 100. The endoprosthesis can be separated into first and second portions longitudinally rather than axially, and can include additional sections. Endoprosthesis 100 is made up of composite 106 which includes a first structure 107 having portions 108, 109, extending into both the first portion 102 and the second portion 104 of the endoprosthesis, respectively. First structure 108 includes interconnected portions 110, 112 that define channels 114, which are open to the exterior of the first structure. Second material 120 is in the channels of the portion 108 of first structure 107 that extends into the first portion 102 of endoprosthesis 100. A layer 122 of second material 120 also encapsulates the interior-facing surface of the portion 108 of first structure 107 that extends into the first portion of the endoprosthesis. Channels of the portion 109 of first structure 107 that extends into the second portion 104 of the endoprosthesis remain substantially open or unfilled. Such open channels may encourage tissue ingrowth once the endoprosthesis has been implanted or deployed, which may advantageously stabilize the endoprosthesis in vivo. The layer of second material may serve as a barrier to prevent further tissue ingrowth into the lumen of the endoprosthesis.

Another embodiment is illustrated in Fig. 6, in which a generally cylindrical endoprosthesis 200 includes a first portion 202 which makes up the interior portion of the endoprosthesis and a second portion 204 which makes up the outer portion of the endoprosthesis. Endoprosthesis 200 includes composite 206 which has a first structure 207 having portions 208, 209, extending into both the first portion 202 and the second portion 204 of the endoprosthesis, respectively. First structure 208 includes interconnected portions 210, 212 that define interconnected channels 214, which are open to the exterior of the first structure. Second material 220 is in the channels 214 of the portion 208 of first structure 207 that extends into the first portion 202 of endoprosthesis 200. A layer 222 of second material 220 also encapsulates the interior-facing surface of the portion 208 of first structure 207 that extends into the first portion of the endoprosthesis. A third material 230 is in the channels 214 of the portion 209 of the first structure 207 that extends into the second portion 204 of endoprosthesis 200. A layer 232 of third material encapsulates the exterior-facing surface of the portion 209 of first structure 207 that extends into the second portion 204 of endoprosthesis 200. Such a design may allow a first structure comprised of a material which does not have proven biocompatibility to be utilized by permitting the unproven material to be fully encapsulated with second and third material, each of which may be biocompatible, thus rendering the endoprosthesis biocompatible. Without permanent and complete encapsulation, only biocompatible materials with long, successful implant clinical experience are typically used. With the encapsulation, materials with unique properties but without implant clinical experience can be used (e.g., copper for MRI magnetic susceptibility compatibility, silver for MRI magnetic susceptibility compatibility and radiopacity enhancement, tungsten for high stiffness and radiopacity enhancement). In some embodiments, one or more of the first, second and third materials may be biodegradable and/or bioabsorbable. Such may allow for encapsulation of the endoprosthesis, for example, with a layer of biodegradable third material to provide a smooth outer endoprosthesis surface, which may reduce or prevent damage to the lumen through which it is passed prior to deployment, while also allowing for the channels of the first structure that are open to the exterior of the endoprosthesis to become exposed and empty, allowing for tissue ingrowth as described above.

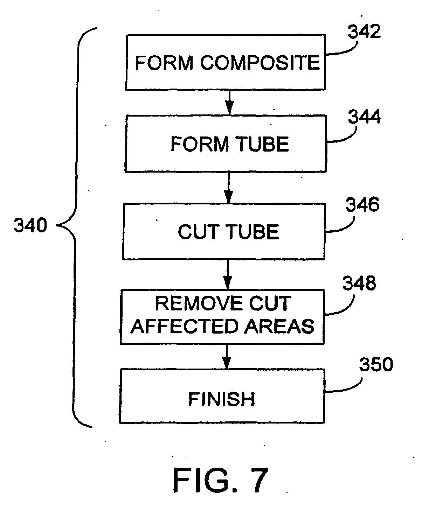

Fig. 7 shows a method 340 of making stent 20. As shown, method 340 includes forming a composite (step 342) and using the composite to form a tube (step 344) that makes up the tubular member of stent 20. The tube is subsequently cut to form bands 22 and connectors 24 (step 346) to produce an unfinished stent. Areas of the unfinished stent affected by the cutting may be subsequently removed (step 348). The unfinished stent may be finished to form stent 20 (step 350).

The steps of forming the composite (step 342) and of using the composite to form a tube (step 344) can optionally take place as a single step, in which the composite is formed in the shape of a tube as the composite is put together. Also, the steps of cutting the tube and removing the cut affected areas may be eliminated, for example, where the composite is formed in the final shape or form of the endoprosthesis (e.g., is formed in a stent-shaped mold).

In certain embodiments, a first structure is formed as an initial step. The first structure in certain embodiments is made by vacuum plasma spray deposition in which the structural metal in powder form is passed through a plasma torch and the droplets splat down onto a substrate. The porosity level in the deposit can be controlled by the powder particle size, gas flow rate into the nozzle, and impingement angle of the spray plume upon the substrate. The porous deposit can be built up to significant thicknesses if desired (e.g., up to one inch thick).

In certain embodiments, a wire mesh first structure is made by weaving or braiding individual wires to form a gridwork pattern. The interstitial open space within the braid or weave can be controlled by the number of wires used (more wires leave smaller openings), the thickness of the wire used, and the number of crossover points. Wires can be chopped into short fibers and tumbled, causing them to bend about each other to form an entanglement. The tangled wires can be mechanically attached to each other, leaving open spaces between the wires. In certain embodiments, metal rings with a spiral, "S"-shaped, "C"-shaped or hook-shape can be interlocked to form a tangled metal structure, either in place of or in conjunction with the use of wires. The rings can be formed of chopped tubing or can be produced from sheet/foil that has been cut and mechanically formed. Metal turnings can be machined off of a bar or plate. Turning size can be tailored by the dimensions of the bar and/or the dimensions of the tooling used during machining and/or the amount of material removed. The shapes can be mechanically combined (tangled) by shaking, vibrating, tumbling, or hand or machine assembly. The preforms made by this technique can be chains, agglomerates, bunches, or bricks.

In certain embodiments, a first structure in the form of a honeycomb is formed by making holes in a flat strip of material, e.g., by drilling or chemical machining. The flat strips can optionally be made to be wavy or rippled, e.g., using metal press forming equipment. The formed strips can then be brazed, welded, or mechanically attached together to form a honeycomb structure.

Instead of using wires to make a tangled wire structure, metal rings with a spiral, "S"-shaped, "C"-shaped or hook-shape can be interlocked to form a tangled metal structure. They can be from chopped tubing or from sheet/foil that has been cut and mechanically formed. Metal turnings can be machined off of a bar or plate. Turning size can be tailored by the amount of material removed and/or the dimensions of the bar and/or the dimensions of the tooling used during machining. The shapes can be mechanically combined (tangled) by shaking, vibrating, tumbling, or hand assembly. The preforms made by this technique can be chains, agglomerates, bunches, or bricks.

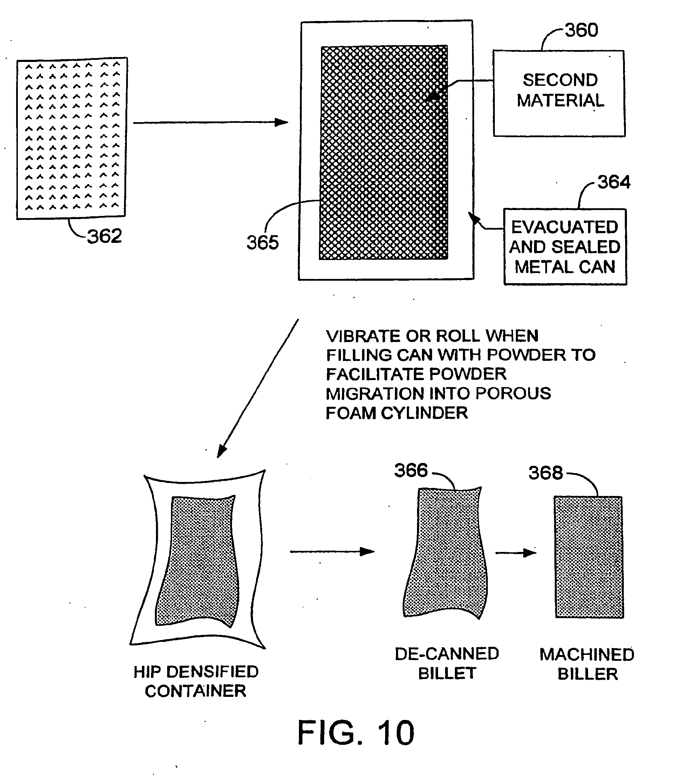

The composite formed in step 342 includes a first structure comprising a first material and having interconnected portions that define interconnected channels, and a second material located in at least some of the channels, wherein the first material and second material have different chemical compositions. In some embodiments, for example, the embodiment illustrated in Fig. 10, a dry composite 365 is formed by placing a first structure 362 into a metal can 364 and introducing a second material 360 in powdered form into the can 364 to fill at least some of the channels in the first structure 362. The dry composite 365 is then densified, consolidated or compressed under pressure to form a composite 366. The second material has a powder particle size smaller than the minimum width of the channels (e.g., no more than about 300 microns, no more than about 100 microns, no more than about 50 microns, no more than about 10 microns) to permit the material to enter and move through the channels. Optionally, the first structure is agitated, e.g., shaken, vibrated or centrifugally rotated, during or subsequent to introduction of the powdered second material to assist with introduction of and distribution of the powder throughout the first structure. Consolidation of the dry composite may be performed by cold pressing, sintering, or diffusion bonding, e.g., hot isostatic pressing, the dry composite. Such consolidation may pack the materials into denser masses, for example, may consolidate the dry composite into a component approaching 100% theoretical density. The composite 366 is then milled or machined to form a billet 368.

The composite preform need not be fully dense, such as, for example, if it is to be processed by additional shapeforming operations that will increase density. For example, a preform that is 50% or more dense can be hot extruded or hot bar rolled to decrease diameter and increase length; the compressive forming may close the voids in the composite and increase the density. In some embodiments, the final endoprosthesis may have a density less than about 100% theoretical density (e.g., less than about 99% theoretical density, less than about 95% theoretical density, less than about 90% theoretical density, less than about 85% theoretical density, or less than about 80% theoretical density). An endoprosthesis having a density below about 100% theoretical density may have porosity that can be utilized, for example, for carrying therapeutic agents or can be utilized to facilitate implant anchoring via tissue ingrowth. For example, a stent with a solid, continuous strut inner surface and a porous (less than about 100% theoretical density) strut outer surface may allow tissue to grow into the stent structure to facilitate anchoring of the strut, while not allowing tissue ingrowth through the stent struts and into the lumen. The porous outer strut material is in certain embodiments infiltrated with a substance that may promote fast tissue ingrowth so that the stent can be expanded into the vessel wall with less pressure and less injury, as some of the anchoring may be accomplished by rapid tissue ingrowth into the porous outer material.

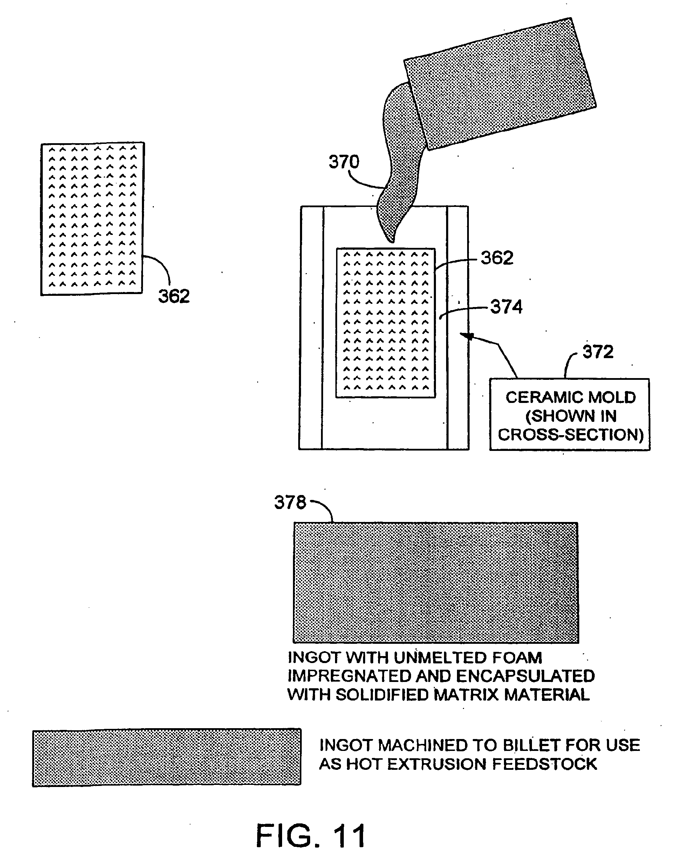

In certain embodiments, molten second material is introduced into at least some of the channels in the first structure to form the composite. For example, in the method illustrated in Fig. 11, a first structure 362 is placed into a ceramic mold 372, and molten second material 370 is poured into the mold to impregnate the channels in the first structure 362 and form the composite 378. A gap 374 exists between the first structure 362 and the interior walls of the ceramic mold 372 to permit the molten second material 372 to cover the exterior of and encapsulate the first structure 362. The second material may be melted by vacuum induction skull method, which allows the molten alloy to be contained within a thin solid shell of the same composition, which can be advantageous when the alloy is highly reactive, e.g., titanium. The second material may optionally have a lower melting temperature than the first material, e.g., the second material may be titanium (which has a melting point of 1660 °C) and the first structure may be tantalum (which has a melting point of 2996 °C). The first structure may be placed in a mold, and second material may be introduced to the mold and allowed to flow into at least some of the channels in the first structure, optionally with the aid of pressure. The first structure may be positioned in the mold such that some or all of the exterior surfaces of the first structure are covered by or encapsulated with second material. Optionally, the molten second material may be introduced into at least some of the channels in the first structure by introducing, e.g., dipping, the first structure may into molten second material, or by vacuum plasma spraying the molten second material into the first structure, either of which does not require a mold.

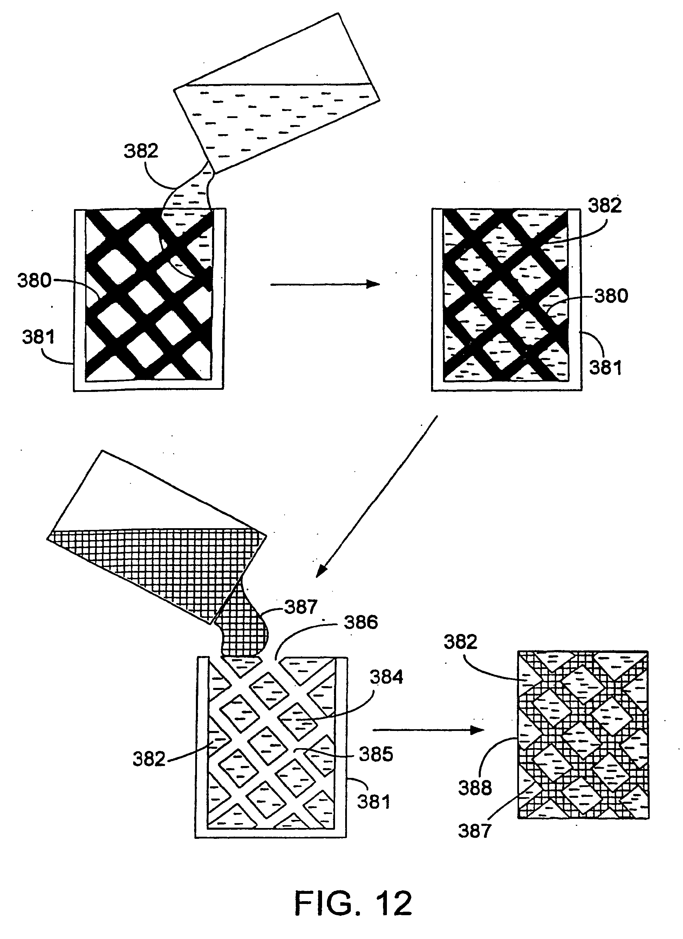

In certain embodiments, as illustrated in Figure 12, the composite is formed by creating a mold out of second material, the mold having hollow passages in the shape of the first structure, and introducing molten first material into the hollow passages, again optionally with the aid of pressure. A core structure 380, having the form desired for the first structure, is placed in a container 381. Molten second material 382 is introduced into the container and allowed to harden. The core structure 380 is then removed, leaving a hardened second material mold 383 having hollow channels or passages 385 where the core structure had been. The channels have at least one opening 386 at a face of hardened second material mold 383 into which a molten first material 387 is introduced forming a composite 388. The first material 387 is allowed to cool and harden. The core structure 380 is made of material that can be removed from the second material, for example, by ashing, leaching, dissolving or the like (e.g., a ceramic which may be removed from the second material, for example, by leaching). The core structure extends to the edge of the mold in at least one location to provide an exposed or open channel, upon having removed the core structure, to permit introduction of first material. A third material having a different chemical composition than the second material can be incorporated, either as a part of the mold or as a part of the first structure. The first material may be added as a powder rather than in molten form in a fashion similar to that described above.

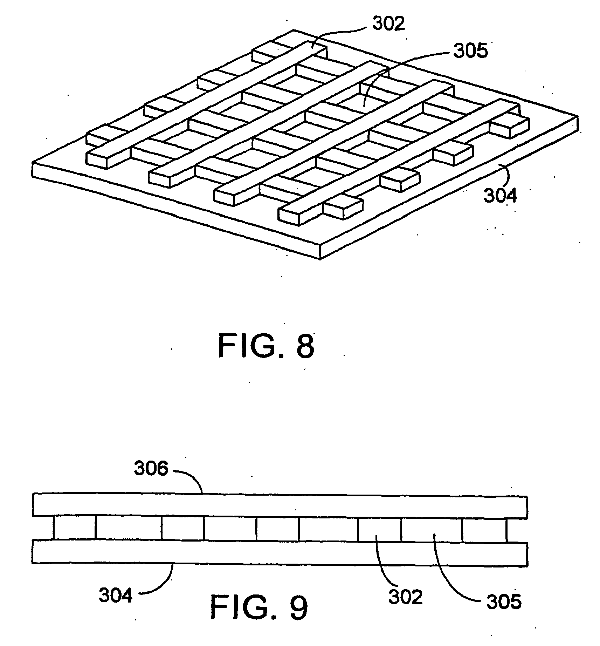

In some embodiments, for example those illustrated in Fig. 8, the composite is made from a first material having a first structure in the form of a mat 302. The mat 302 is overlayed with a layer of second material 304, and the mat and the second material are bonded, for example, by annealing or diffusion bonding, such that at least some second material enters at least one channel 305 in the mat 302. As illustrated in Fig. 9, a layer of third material 306 may also be included, which may overlay a side of the mat opposite that of the layer of second material. Such a third material may have a different chemical composition than the second material, the first material, or both. In such a fashion, an endoprosthesis, e.g., a stent, can be formed having different materials on first and second portions of the stent, for example, on an outer portion of the stent and an inner portion of the stent. This allows for the formation of an endoprosthesis having different materials at the exterior surface and interior surface mechanically and/or metallurgically held together where such materials exhibit poor solid solubility with each other or are difficult to process into single-phase, homogeneous solid solutions with each other such that they may risk delamination absent the first structure. For example, tantalum and titanium typically are not alloyed at more than a couple weight percent with austenitic stainless steel, such as 316L, because these elements are not austenite phase stabilizers; other phases such as ferrite or martensite can form in the titanium or tantalum-bearing alloy which can render the alloy more magnetic than 316L, thereby posing a safety risk with MRI. As another example, it may be desirable to combine iridium with titanium to increase the relatively low radiopacity and modulus of titanium, however, only up to about 10 atomic percent iridium, likely not enough to significantly enhance the radiopacity and modulus of the alloy over that of pure titanium, can be added to titanium without forming brittle intermetallic phases. As yet another example, tantalum and titanium are difficult to alloy together by melting, because the melting temperature for tantalum is much higher than for titanium. The titanium may tend to solidify before the tantalum, resulting in significant chemical heterogeneity in the ingot. This can lead to tearing during forming and non-uniform mechanical properties in the final endoprosthesis.

In certain embodiments, the third material may be the same as the second material, for example, to permit the formation of a stent that is fully encapsulated by a second material. Li a similar fashion, bands of second and third material can be overlayed on the mat to provide an endoprosthesis having different composites longitudinally. Such may allow, for example, for the inclusion of a band of material exhibiting desired RO properties to act as a radiopaque marker on the endoprosthesis. Similarly, the first structure can include discrete layers of first material and an additional material differing in chemical structure from the first material.

In some embodiments, a layer of second material can have a first structure imposed upon a surface by vapor deposition. For example, a thin-walled stent tube made up of second material can be drawn and used as a solid inner layer of a stent, and a first structure can be grown on the exterior layer to form the first structure.

After the composite is formed, it is formed into a tube (step 344). In some embodiments, a billet is created from the composite, for example, by cold isostatic pressure, hot isostatic pressure, or sintering of the composite. The billet may then be formed into a rod shape, for example, by hot extrusion or hot rolling of the billet. The rod may then be gun drilled, that is, have a bore drilled longitudinally down the center of the rod, to form a hollow rod, which may then be drawn, e.g., cold drawn, to form tubing in about the desired configuration for the prosthesis. Alternately, metal injection molding can be used to produce bar, tubing, or stent tubing blanks at near net size.

Alternatively or additionally, other thermomechanical processes can also be used to form a tubular member made of a mechanical composite material. For example, the second material need not be molten to form the composite material. The second material and the first structure can be combined by powder metallurgy techniques (such as pressure casting, sintering, hot isostatic pressing, and hot working), slurry mixing, direct laser sintering, and vacuum plasma deposition, to form a raw material that is subsequently shaped into a feedstock, such as a hollow tubular member. A medical device including a composite material having variable concentrations of first and second (and, where applicable, third, fourth, etc.) materials can be made by joining multiple portions (e.g., billets) of composites having different concentrations by sintering. Endoprostheses, e.g. stents, with layers of composite of different concentrations can be formed by sequentially adding the selected composites into a mold to form the tubular member. hi some embodiments, the hollow tubular member including the composite can be drawn through a series of dies with progressively smaller circular openings to plastically deform the member to a targeted size and shape. The plastic deformation strain can harden the member (and increases its yield strength) and elongate grains of some or all materials used to form the composite along the longitudinal axis of the member. The deformed member can be heat treated (e.g., annealed above the recrystallization temperature and/or hot isostatically pressed) to transform the elongated grain structure into an initial grain structure, e.g., one including equiaxed grains. Small or fine grains can be formed by heating the member close to the recrystallization temperature for a short time. Large or coarse grains can be formed by heating the member at higher temperatures and/or for longer times to promote grain growth.

Once in tubing form, bands 22 and connectors 24 of stent 20 are formed, as shown, by cutting the tube (step 346). Selected portions of the tube can be removed to form bands 22 and connectors 24 by laser cutting, as described in U.S. Patent No. 5,780,807. In certain embodiments, during laser cutting, a liquid carrier, such as a solvent or an oil, is flowed through the lumen of the tube. The carrier can prevent dross formed on one portion of the tube from re-depositing on another portion, and/or reduce formation of recast material on the tube. Other methods of removing portions of the tube can be used, such as mechanical machining (e.g., micro-machining), electrical discharge machining (EDM), and photoetching (e.g., acid photoetching).

In some embodiments, after bands 22 and connectors 24 are formed, areas of the tube affected by the cutting operation above can be removed (step 348). For example, laser machining of bands 22 and connectors 24 can leave a surface layer of melted and resolidified material and/or oxidized metal that can adversely affect the mechanical properties and performance of stent 20. The affected areas can be removed mechanically (such as by grit blasting or honing) and/or chemically (such as by etching or electropolishing). In some embodiments, the tubular member can be near net shape configuration after step 348 is performed. "Near-net size" means that the tube has a relatively thin envelope of material that is removed to provide a finished stent. In some embodiments, the tube is formed less than about 25% oversized, e.g., less than about 15%, 10%, or 5% oversized.

The unfinished stent is then finished to form stent 20 (step 350). The unfinished stent can be finished, for example, by electropolishing to a smooth finish. Since the unfinished stent can be formed to near-net size, relatively little of the unfinished stent need to be removed to finish the stent. As a result, further processing (which can damage the stent) and costly materials can be reduced. In some embodiments, about 0.0001 inch of the stent material can be removed by chemical milling and/or electropolishing to yield a stent.

Stent 20 can be of a desired shape and size (e.g., coronary stents, aortic stents, peripheral vascular stents, gastrointestinal stents, urology stents, and neurology stents). Depending on the application, stent 20 can have a diameter of between, for example, 1 mm to 46 mm. m certain embodiments, a coronary stent can have an expanded diameter of from about 2 mm to about 6 mm. hi some embodiments, a peripheral stent can have an expanded diameter of from about 5 mm to about 24 mm. hi certain embodiments, a gastrointestinal and/or urology stent can have an expanded diameter of from about 6 mm to about 30 mm. hi some embodiments, a neurology stent can have an expanded diameter of from about 1 mm to about 12 mm. An abdominal aortic aneurysm (AAA) stent and a thoracic aortic aneurysm (TAA) stent can have a diameter from about 20 mm to about 46 mm. Stent 20 can be balloon-expandable, self-expandable, or a combination of both (e.g., U.S. Patent No. 5,366,504).

In use, stent 20 can be used, e.g., delivered and expanded, using a catheter delivery system. Catheter systems are described in, for example, Wang U.S. 5,195,969, Hamlin U.S. 5,270,086, and Raeder-Devens, U.S. 6,726,712. Stents and stent delivery are also exemplified by the Radius® or Symbiot® systems, available from Boston Scientific Scimed, Maple Grove, MN.

While a number of embodiments have been described above, the invention is not so limited.

As an example, while stent 20 is shown above as being formed wholly of composite 26, in other embodiments, the composite forms one or more selected portions of the medical device. For example, stent 20 can include multiple layers in which one or more layers include a composite, and one or more layers do not include a composite. The layer(s) that includes a composite can include the same composite materials or different composite materials. The layer(s) that does not include a composite may include one or more of the biocompatible materials listed above. The layering of the composite material provides yet another way to tailor and tune the properties of the medical device. Stents including multiple layers are described, for example, in published patent application 2004-0044397, and Heath, U.S. 6,287,331.

Stent 20 can be a part of a covered stent or a stent-graft. In other embodiments, stent 20 can include and/or be attached to a biocompatible, non-porous or semi-porous polymer matrix made of polytetrafluoroethylene (PTFE), expanded PTFE, polyethylene, urethane, or polypropylene.

Stent 20 can include a releasable therapeutic agent, drug, or a pharmaceutically active compound, such as described in U.S. Patent No. 5,674,242, U.S.S.N. 09/895,415, filed July 2, 2001, and U.S.S.N. 10/232,265, filed August 30, 2002. The therapeutic agents, drugs, or pharmaceutically active compounds can include, for example, anti-thrombogenic agents, antioxidants, anti-inflammatory agents, anesthetic agents, anticoagulants, and antibiotics.

In some embodiments, stent 20 can be formed by fabricating a wire including the composite, and knitting and/or weaving the wire into a tubular member.

The endoprosthesis may include a plurality of composites having different compositions.

The following examples are illustrative and not intended to be limiting.

### Example 1

A titanium matrix can be cast about a tantalum porous structure. Tantalum wire mesh such as Goodfellow (Huntingdon, England), part number TA008710 (order code 349-745- 35), with a nominal wire diameter of 0.075 mm and 72% open area, can be stacked to form an assembly that is about 1 mm thick. The assembly can be suspended in the middle of a 3 mm cavity in a ceramic mold. Titanium can be melted via e-beam, plasma, or induction skull melting and poured into the mold. Since the titanium melts at 1668C and the tantalum melts at 2996C, the tantalum will remain solid as the liquid titanium flows into the open areas of the mesh and encapsulates the tantalum mesh assembly. After solidification and cooling, the ceramic mold can be dissolved or broken away from the metal casting. The three mm-thick casting can be canned (encapsulated) with steel or stainless steel, evacuated, and sealed. The container can be hot isostatically pressed at 2150F for 8 hours in order to densify the casting. The hot isostatically pressed casting can then be hot or cold rolled with interpass anneals at 700C to a thickness of 0.10 mm. The strip can then be rolled into a tube and TIG seam welded. The welded tube can then be mandrel drawn to 0.072 outer diameter and 0.075 mm wall thickness to cold work the weld thereby making the weld stronger. A coronary stent can be cut from the drawn or heat treated tube using laser machining technique. The cut stent can be electropolished to final dimensions and surface finish. The result will be a stent with a surface consisting primarily of titanium and with some intersections of tantalum from the core to the surface and a core of tantalum lacing through the titanium matrix. The tantalum will provide radiopacity and stiffness enhancement, since it has higher density and modulus than titanium, and the stent will have high compression resistance than a comparable stent formed of pure talantum, because titanium has a higher strength than tantalum.

### Example 2

Alternate layers of tantalum mesh from the previous example and 1 mm thick Ti 6A1- 4V foil (Goodfellow TI010500) can be overlayed until the assembly is about 10 mm thick, with the bottom and top layers being titanium alloy foil. Two additional layers of the titanium alloy foil can then be added to the top and bottom of the stack. The titanium layers should extend out 25 mm beyond all tantalum mesh edges. The edges of the titanium layers can be tack welded to hold the stack together. The stack can then be hot or cold pressed to achieve a 50-70% reduction in thickness. The pressed preform can then be alternately cold rolled and annealed until a thickness of 0.10mm is achieved. The strip can then be rolled into a tube and seam welded. The welded tube can then be mandrel drawn to 0.075 mm wall thickness. A stent can be cut from the drawn or heat treated tube using laser machining technique. The cut stent can be electropolished to final dimensions and surface finish. The result will be a stent with a surface consisting primarily of titanium and with some intersections of tantalum from the core to the surface and a core of tantalum lacing through the titanium matrix.

### Example 3

A tantalum wire mesh assembly, e.g., as in the first two examples, can be placed within a tubular ceramic mold and cast with titanium or interlayered between coaxial titanium tubes such that cylindrical preforms can be made. The preforms may then be mandrel or floating plug tube drawn with interpass anneals to form stent tubing.

### EXAMPLES

The following examples are provided to help understanding the invention:
1. An endoprosthesis comprising a composite, the composite comprising a first structure comprising a first material and having interconnected portions that define interconnected channels, and a second material in at least some of the interconnected channels, the first material and second material having different chemical compositions.
2. The endoprosthesis of example 1 , wherein the first material and the second material differ from each other in at least one of the following properties:
   (a) radiopacity;
   (b) magnetic susceptibility;
   (c) yield strength;
   (d) ductility; and
   (e) stiffness.
3. The endoprosthesis of example 1 , wherein at least about 50% of the channels defined by the interconnected portions of the first structure are interconnected.
4. The endoprosthesis of example 1 , wherein the endoprosthesis is made from a preform formed of the composite, and at least about 50% of the channels defined by the interconnected portions of the first structure are interconnected.
5. The endoprosthesis of example 1, wherein at least about 75% of the channels defined by the interconnected portions of the first structure are interconnected.
6. The endoprosthesis of example 1 , wherein the first material comprises a metal or an alloy.
7. The endoprosthesis of example 1 , wherein the second material comprises a metal or an alloy.
8. The endoprosthesis of example 1 , wherein one of the first material and second material comprises a biodegradable polymer.
9. The endoprosthesis of example 1 , wherein substantially all of the channels defined by the interconnected portions of the first structure connected to at least one channel that is open to the exterior of the first structure.
10. The endoprosthesis of example 1 , wherein the first structure comprises a shape selected from the group consisting of a foam, a wire weave mesh, a plasma-sprayed deposit, a braided wire, a tangled wire, a honeycomb and combinations thereof.
11. The endoprosthesis of example 1 , wherein between about 10% and about 90% of the total volume of the first structure comprises channels.
12. The endoprosthesis of example 11 , wherein between about 50% and about 90% of the total volume of the first structure comprises channels.
13. The endoprosthesis of example 1 , wherein the channels are between 10 microns and 10 mm in size at their narrowest part.
14. The endoprosthesis of example 1 , wherein the first structure has a maximum width that is less than the width of a side the endoprosthesis.
15. The endoprosthesis of example 14, wherein the maximum width of the skeletal structure is between 5% and 50% of the width of the endoprosthesis.
16. The endoprosthesis of example 1, wherein the first structure is substantially encapsulated by the second material.
17. The endoprosthesis of example 1 , wherein the second material is bonded to the first structure.
18. The endoprosthesis of example 17, wherein the second material is mechanically bonded to the first structure.
19. The endoprosthesis of example 17, wherein the second material is metallurgically bonded to the first structure.
20. The endoprosthesis of example 1, wherein the second material has a higher density than the first material.
21. The endoprosthesis of example 1 , wherein one of the first material and the second material comprises a material selected from the group consisting of 316L stainless steel, cobalt and alloys thereof, titanium and alloys thereof, and nickel and alloys thereof.
22. The endoprosthesis of example 1, wherein one of the first material and the second material comprises a material selected from the group consisting of tantalum, rhenium, indium, platinum, gold, silver, iridium, niobium, and alloys of any of these.
23. The endoprosthesis of example 1 , wherein one of the first material and the second material comprises a material selected from the group consisting of molybdenum, tungsten, chromium, tantalum over carbon Trabecular Metal™ foam, and niobium over carbon Trabecular Metal™ foam.
24. The endoprosthesis of example 1, wherein one of the first material and the second material comprises a material selected from the group consisting of 316L stainless steel, L605, MP35N® alloy, Elgiloy®, platinum enhanced radiopaque stainless steel (PERSS), Nb-IZr, Nioballoy, NiTi, Biodur® 108 stainless steel, zirconium, an alloy of zirconium, titanium, an alloy of titanium, biostable polymer and bioabsorbable polymer.
25. The endoprosthesis of example 1, wherein the endoprosthesis is a stent.
26. The endoprosthesis of example 1, wherein the endoprosthesis is a stent graft.
27. The endoprosthesis of example 1, wherein second material is in at least 50% of the interconnected channels defined by the first structure.
28. An endoprosthesis having a first portion and a second portion, wherein the endoprosthesis comprises a composite having: a first structure comprising a first material and having interconnected portions that define interconnected channels, the first structure having portions that extend into both the first portion of the endoprosthesis and the second portion of the endoprosthesis; and an second material in at least some of the interconnected channels of the portion of the first structure that extends into the first portion of the endoprosthesis, wherein the first material has a different chemical composition than the second material.
29. The endoprosthesis of example 28, wherein the first material and the second material differ from each other in at least one of the following properties:
   (a) radiopacity;
   (b) magnetic susceptibility;
   (c) yield strength;
   (d) ductility; and
   (e) stiffness.
30. The endoprosthesis of example 28, wherein least about 50% of the channels defined by the interconnected portions of the first structure are interconnected.
31. The endoprosthesis of example 28, wherein at least about 75% of the channels defined by the interconnected portions of the first structure are interconnected.
32. The endoprosthesis of example 28, wherein the first portion of the endoprosthesis is disposed inwardly relative to the second portion of the endoprosthesis.
33. The endoprosthesis of example 28, wherein the composite further comprises a third material in at least some of the interconnected channels of the portion of the first structure that extends into the second portion of the endoprosthesis.
34. The endoprosthesis of example 33, wherein the first material, second material and third material have chemical compositions different from each other.
35. The endoprosthesis of example 33, wherein the second material has poor solid solubility with the third material.
36. The endoprosthesis of example 33, wherein second material is in at least 50% of the interconnected channels defined by the portion of the first structure that extends into the second portion of the endoprosthesis.
37. The endoprosthesis of example 28, wherein second material is in at least 50% of the interconnected channels defined by the portion of the first structure that extends into the first portion of the endoprosthesis.
38. A method of making an endoprosthesis, the method comprising: introducing a second material into at least some interconnected channels defined by interconnected portions of a first structure comprising a first material to form a composite, the first and second materials having different chemical compositions, and using the composite to form at least a portion of the endoprosthesis.
39. The method of example 38, wherein powdered second material is introduced into at least some of the interconnected channels in the first structure to form a dry composite.
40. The method of example 38, further comprising agitating the first structure to distribute the powdered second material.
41. The method of example 40, wherein the step of agitating comprises shaking, vibrating or centrifugally rotating the first structure.
42. The method of example 38, further comprising consolidating the dry composite under pressure.
43. The method of example 42, wherein the step of consolidating is performed by cold pressing, sintering, or hot isostatic pressing.
44. The method of example 38, wherein molten second material is introduced into at least some of the interconnected channels in the first structure.
45. The method of example 44, wherein the second material has a lower melting temperature than the first structure.
46. The method of example 44, further comprising melting the second material by vacuum induction skull method prior to introducing it into at least some of the interconnected channels in the first structure.
47. The method of example 44, wherein the second material comprises titanium and the first structure comprises tantalum.
48. The method of example 44, wherein the first structure is placed in a mold and molten second material is introduced into the mold.
49. The method of example 38, wherein the composite is formed by creating a mold out of first material, the mold having interconnected portions defining interconnected channels, and introducing molten second material into the interconnected channels.
50. The method of example 49, wherein the mold is created by introducing molten first material into a mold having a ceramic core in the form of the interconnected channels and allowing the first material to harden, wherein the ceramic core extends to at least one edge of the mold; removing the ceramic core to leave the interconnected in the hardened first material.
51. The method of example 38, wherein the composite is formed by forming a first structure in the form of a mat, overlaying the mat with a layer of second material, and bonding the mat to the second material.
52. The method of example 51, wherein the mat is bonded to the second material by annealing or diffusion bonding.
53. The method of example 51, further comprising overlaying the mat with a layer of third material.
54. The method of example 53, wherein the layer of third material is on an opposite face of the mat from the layer of second material.
55. The method of example 53, wherein the third material has a different chemical composition than the second material.
56. The method of example 38, further comprising densifying the composite into a billet.
57. The method of example 56, wherein the composite is densified by applying heat and isostatic pressure to the composite.
58. The method of example 56, further comprising hot pressing, hot extruding or forging the billet to form a rod, gun drilling the rod, drawing the drilled rod into tubing and laser cutting the tubing to form the endoprosthesis.
59. The method of example 58, further comprising coating the endoprosthesis with a biocompatible material.
60. The method of example 38, wherein second material is introduced into at least 50% of the interconnected portions of the first structure.

## Claims

1. An endoprosthesis comprising a composite, the composite comprising
a first structure (28;40) comprising a first material (29) and having interconnected portions (31;42) define interconnected channels (30;40), and
a second material (34) in at least some of the interconnected channels (30) the first material (29) and second material (34) having different chemical compositions, wherein the second material (34) comprises a metal or metal alloy and wherein the first structure (28;40) is substantially encapsulated by the second material.

2. The endoprosthesis of claim 1, wherein the first material (29) comprises a biodegradable polymer.

3. The endoprosthesis of claim 1, wherein at least 50% of the channels defined by the interconnected portions (31;42) of the first structure (28;40) are interconnected, preferably wherein at least 75% of the channels defined by the interconnected portions of the first structure (28;40) are interconnected.

4. The endoprosthesis of claim 1, wherein between about 10% and 90% of the total volume of the first structure (28;40) comprises channels, preferably wherein between about 50% and 90% of the total volume of the first structure comprises channels.

5. The endoprosthesis of claim 1, wherein the first material (29) comprises a metal or metal alloy.

6. The endoprosthesis of claim 1, wherein the second material (34) has a higher density than the first material.

7. The endoprosthesis of claim 1, wherein the second material (34) comprises a material selected from the group consisting of 316L stainless steel, cobalt and alloys thereof, titanium and alloys thereof, nickel and alloys thereof; or wherein the second material comprises a material selected from the group consisting of tantalum, rhenium, indium, platinum, gold, silver, iridium, niobium, and alloys of these; or wherein the second material comprises a material selected from the group consisting of molybdenum, chromium, tantalum over carbon Trabecular MetalTM foam, and niobium over carbon Trabecular MetalTM foam.

8. The endoprosthesis of claim 1, wherein the endoprosthesis is a stent or a stent graft.

9. The endoprosthesis of claim 1, wherein the second material (34) is in at least 50% of the channel defined by the first structure.

10. The endoprosthesis of claim 1, wherein the channels are between 10 microns and 10 mm in their narrowest part.

11. The endoprosthesis of claim 1, wherein the second material (34) is bonded to the first structure, preferably wherein the second structure is mechanically bonded to the first structure.

12. A method for making an endoprosthesis, the method comprising:
introducing a second material (34) into at least some interconnected channels (30)defined by interconnected portions of a first structure (28;40) comprising a first material (29) to form a composite,
wherein the second material (34) comprises a metal or an alloy, the first and second material having different chemical compositions, and wherein the second material (34) encapsulates the first material (29), and
using the composite to form at least a portion of the endoprosthesis.

13. The method of claim 12, wherein the first material (29) comprises a biodegradable polymer.

14. The method of claim 12, wherein powdered second material (34) is introduced into at least some of the interconnected channels in the first structure to form a dry composite.

15. The method of claim 14, further comprising consolidating the dry composite under pressure, preferably wherein the step of consolidating is performed by cold pressing, sintering, or hot isostatic pressing.

## Patentansprüche

1. Eine Endoprothese, die ein Gemisch umfasst, wobei das Gemisch umfasst:
eine erste Struktur (18; 40), die ein erstes Material (29) mit verbundenen Bereichen (31; 42) die verbundene Kanäle (30; 40) definieren, umfasst, und
ein zweites Material (34) in zumindest einigen der verbundenen Kanäle (30), wobei das erste Material (29) und das zweite Material (34) verschiedene chemische Zusammensetzungen aufweisen, wobei das zweite Material (34) ein Metall oder eine Legierung umfasst und wobei die erste Struktur (28; 40) im Wesentlichen eingekapselt ist durch das zweite Material.

2. Die Endoprothese gemäß Anspruch 1, wobei das erste Material (29) ein biodegradierbares Polymer umfasst.

3. Endoprothese gemäß Anspruch 1, wobei zumindest 50% der Kanäle, die durch die verbundenen Bereiche (31; 42) der ersten Struktur (28; 40) definiert werden, verbunden sind, vorzugsweise wobei zumindest 75% der Kanäle, die durch die verbundenen Bereiche der ersten Struktur (28; 40) definiert werden, verbunden sind.

4. Endoprothese gemäß Anspruch 1, wobei zwischen ungefähr 10% und 90% des Gesamtvolumens der ersten Struktur (28; 40) Kanäle umfasst, vorzugsweise wobei zwischen ungefähr 50% und 90% des Gesamtvolumens der ersten Struktur Kanäle umfasst.

5. Endoprothese gemäß Anspruch 1, wobei das erste Material (29) ein Metall oder eine Legierung umfasst.

6. Endoprothese gemäß Anspruch 1, wobei das zweite Material (34) eine höhere Dichte hat als das erste Material.

7. Endoprothese gemäß Anspruch 1, wobei das zweite Material (34) ein Material umfasst ausgewählt aus der Gruppe bestehend aus 316L Edelstahl, Kobalt und Legierungen davon, Titan und Legierungen davon, Nickel und Legierungen davon; oder wobei das zweite Material ein Material umfasst ausgewählt aus der Gruppe bestehend aus Tantal, Rhenium, Indium, Platin, Gold, Silber, Iridium, Niob und Legierungen dieser; oder wobei das zweite Material ein Material umfasst ausgewählt aus der Gruppe bestehend aus Molybdän, Chrom, Tantal über Kohlenstoff Trabecular-Metal^{™}-Schaum, und Niob über Kohlenstoff-Trabecular-Metal^{™}-Schaum.

8. Endoprothese gemäß Anspruch 1, wobei die Endoprothese ein Stent oder ein Stent-Graft ist.

9. Endoprothese gemäß Anspruch 1, wobei das zweite Material (34) sich in mindestens 50% der Kanäle, die durch die erste Struktur definiert werden, befindet.

10. Endoprothese gemäß Anspruch 1, wobei die Kanäle zwischen 10 µm und 10 mm weit sind an ihrer engsten Stelle.

11. Endoprothese gemäß Anspruch 1, wobei das zweite Material (34) mit der ersten Struktur verbunden ist, vorzugsweise wobei die zweite Struktur mechanisch mit der ersten Struktur verbunden ist.

12. Verfahren zum Herstellen einer Endoprothese, das Verfahren umfassend:
Einbringen eines zweiten Materials (34) in zumindest einige verbundene Kanäle (30), die durch verbundene Bereiche einer ersten Struktur (28; 40), die ein erstes Material (29) umfasst, definiert werden, um ein Gemisch zu bilden,
wobei das zweite Material (34) ein Metall oder eine Legierung umfasst, wobei das erste und zweite Material verschiedene chemische Zusammensetzungen haben, und wobei das zweite Material (34) das erste Material (21) einkapselt, und
Benutzen des Gemischs, um zumindest einen Teil der Endoprothese zu bilden.

13. Verfahren gemäß Anspruch 12, wobei das erste Material (29) ein biodegradierbares Polymer umfasst.

14. Verfahren gemäß Anspruch 12, wobei das zweite Material (34) in Pulverform in zumindest einige der verbundenen Kanäle in der ersten Struktur eingeführt wird, um ein trockenes Gemisch zu bilden.

15. Verfahren gemäß Anspruch 14, weiter umfassend Konsolidieren des trockenen Gemisches unter Druck, vorzugsweise wobei der Schritt des Konsolidierens durch Kaltpressen, Sintern oder heißes isostatisches Pressen durchgeführt wird.

## Revendications

1. Endoprothèse comprenant un composite, le composite comprenant
une première structure (28 ; 40) comprenant un premier matériau (29) et ayant des parties interconnectées (31 ; 42) qui définissent des canaux interconnectés (30 ; 40), et
un deuxième matériau (34) dans au moins certains des canaux interconnectés (30), le premier matériau (29) et le deuxième matériau (34) ayant des compositions chimiques différentes, le deuxième matériau (34) comprenant un métal ou un alliage métallique, et la première structure (28 ; 40) étant en grande partie encapsulée par le deuxième matériau.

2. Endoprothèse selon la revendication 1, dans laquelle le premier matériau (29) comprend un polymère biodégradable.

3. Endoprothèse selon la revendication 1, dans laquelle au moins 50 % des canaux définis par les parties interconnectées (31 ; 42) de la première structure (28 ; 40) sont interconnectés, de préférence dans laquelle au moins 75 % des canaux définis par les parties interconnectées de la première structure (28 ; 40) sont interconnectés.

4. Endoprothèse selon la revendication 1, dans laquelle entre environ 10 % et 90 % du volume total de la première structure (28 ; 40) comprend des canaux, de préférence dans laquelle entre environ 50 % et 90 % du volume total de la première structure comprend des canaux.

5. Endoprothèse selon la revendication 1, dans laquelle le premier matériau (29) comprend un métal ou un alliage métallique.

6. Endoprothèse selon la revendication 1, dans laquelle le deuxième matériau (34) a une densité supérieure à celle du premier matériau.

7. Endoprothèse selon la revendication 1, dans laquelle le deuxième matériau (34) comprend un matériau choisi dans le groupe constitué par l'acier inoxydable 316L, le cobalt et ses alliages, le titane et ses alliages, et le nickel et ses alliages ; ou dans laquelle le deuxième matériau comprend un matériau choisi dans le groupe constitué par le tantale, le rhénium, l'indium, le platine, l'or, l'argent, l'iridium, le niobium, et les alliages de ceux-ci ; ou dans laquelle le deuxième matériau comprend un matériau choisi dans le groupe constitué par le molybdène, le chrome, le tantale sur mousse de carbone Trabecular MetalTM, et le niobium sur mousse de carbone Trabecular MetalTM.

8. Endoprothèse selon la revendication 1, l'endoprothèse étant un stent ou un greffon de stent.

9. Endoprothèse selon la revendication 1, dans laquelle le deuxième matériau (34) se trouve dans au moins 50 % du canal défini par la première structure.

10. Endoprothèse selon la revendication 1, dans laquelle les canaux font entre 10 µm et 10 mm dans leur partie la plus étroite.

11. Endoprothèse selon la revendication 1, dans laquelle le deuxième matériau (34) est lié à la première structure, de préférence dans laquelle la deuxième structure est liée mécaniquement à la première structure.

12. Procédé de fabrication d'une endoprothèse, le procédé comprenant :
l'introduction d'un deuxième matériau (34) dans au moins certains canaux interconnectés (30) définis par des parties interconnectées d'une première structure (28 ; 40) comprenant un premier matériau (29) pour former un composite,
dans lequel le deuxième matériau (34) comprend un métal ou un alliage, le premier et le deuxième matériau ayant des compositions chimiques différentes, et dans lequel le deuxième matériau (34) encapsule le premier matériau (29), et
l'utilisation du composite pour former au moins une partie de l'endoprothèse.

13. Procédé selon la revendication 12, dans lequel le premier matériau (29) comprend un polymère biodégradable.

14. Procédé selon la revendication 12, dans lequel le deuxième matériau (34) en poudre est introduit dans au moins certains des canaux interconnectés dans la première structure pour former un composite sec.

15. Procédé selon la revendication 14, comprenant en outre la consolidation du composite sec sous pression, l'étape de consolidation étant de préférence effectuée par pressage à froid, frittage, ou pressage isostatique à chaud.
